Europäisches Patentamt

European Patent Office

Office européen des brevets

(11) Publication number: **0 304 238**
**A2**

## (12) EUROPEAN PATENT APPLICATION

(21) Application number: **88307476.7**

(22) Date of filing: **12.08.88**

(51) Int. Cl.4: **G01N 33/576 , G01N 33/68 ,**
**//G01N33/543**

(30) Priority: **14.08.87 US 85509**

(43) Date of publication of application:
**22.02.89 Bulletin 89/08**

(84) Designated Contracting States:
**BE DE ES FR GB IT LU NL**

(71) Applicant: **ORTHO DIAGNOSTIC SYSTEMS INC.**
**U.S. Route 202**
**Raritan New Jersey 08869(US)**

(72) Inventor: **Nelles, Mitchell J.**
**40 Toby Drive**
**Succasunna New Jersey 07876(US)**
Inventor: **Geltosky, John E.**
**4212 Biddeford**
**Doylestown, PA(US)**
Inventor: **Sito, Alexander F.**
**4846 River Road**
**Point Pleasant, PA 18950(US)**

(74) Representative: **Mercer, Christopher Paul et al**
**Carpmaels & Ransford 43, Bloomsbury**
**Square**
**London WC1A 2RA(GB)**

(54) **Method for detecting and confirming the presence of antibodies.**

(57) A non-competitive method for detecting the presence of Hepatitis B core antibody (anti-HBc) in a biological fluid is provided. The method comprises contacting the fluid with an insoluble surface coated with hepatitis B core antigen (HBcAg). The surface is then contacted with a mixture of a labeled antibody specific for human IgG and a labeled antibody specific for human IgM. Detection of the label bound to the insoluble surface indicates the presence of anti-HBc in the sample. A method for confirming the presence of an antibody of interest in a biological fluid is also provided.

EP 0 304 238 A2

## METHODS FOR DETECTING AND CONFIRMING THE PRESENCE OF ANTIBODIES

Background of the Invention

Throughout this disclosure, various publications and patents are referenced. The disclosures of these publications and patents in their entireties are hereby incorporated by reference into this disclosure in order to more fully describe the state of the art as known to those skilled therein as of the date of the invention described and claimed herein.

This invention relates to methods for detecting and confirming the presence of antibodies of interest in a biological fluid. In particular, this invention relates to a direct, non-competitive immunoassay for detecting antibodies to Hepatitis B core antigen.

Antibody to Hepatitis B core antigen (anti-HBc) has been long recognized to be a sensitive indicator of recent and past Hepatitis B infection. Recently, the presence of anti-HBc in donor blood has been shown to correlate with the incidence of post-transfusion hepatitis (Non A, Non B Hepatitis), which makes the test for anti-HBc also applicable for use as a surrogate test for Non-A, Non-B Hepatitis.

It is desirable to detect anti-HBc antibodies as early as possible in an infected or exposed subject. Anti-HBc antibodies develop early during acute Hepatitis B and persist for long periods in persons convalescing from Hepatitis B infection. Of persons with acute Hepatitis B, 90-95% will have Hepatitis B surface antigen (HBsAg) detectable in their serum when they first present themselves for medical assistance. This leaves 5-10% of acute Hepatitis B undiagnosable by HBsAg testing. If there is a delay in seeking medical assistance, the percentage of undiagnosable acute Hepatitis B cases increases proportionately to the delay. In virtually all acute Hepatitis B cases, however, anti-HBc is present just after the appearance of HBsAg, during the period when HBsAg persists and after HBsAg clears. In addition, anti-HBc is present in virtually all patients with chronic Hepatitis B.

Current commercially available immunoassays for detection of anti-HBc utilize a competitive format in which labeled and unlabeled anti-HBc compete for a limited number of binding sites on an antigen coated solid phase. Problems with competitive assays for detection of anti-HBc are well known. For example, non-specific binding of antibodies in samples lacking anti-HBc may cause a high rate of false positive results (i.e., wrongly diagnosing that a sample contains anti-HBc).

In general, in competitive immunoassays for an antibody of interest, a measured amount of a labeled antibody competes with unlabeled antibody present in a test sample for small amounts of insolubilized antigen. Label activity in the bound or unbound fraction is determined and related by a standard curve to an unknown amount of antibody in the sample. For example, in a competitive enzyme immunoassay a sample which provides an absorbance value lower than a predetermined cutoff value is considered to be reactive for the antibody of interest. Therefore, the color intensity obtained is inversely proportional to the unknown antibody concentration.

A competitive assay for anti-HBc is described in U.S. Patent No. 4,241,175 (Miller et al.) The assay involves a competitive format in which Hepatitis B core antigen (HBcAg) derived from Dane particles is coated on a solid surface. The solid surface is then contacted with a biological fluid suspected of containing anti-HBc and with a competing amount of anti-HBc-enzyme conjugate. U.S. Patent No. 4,016,043 (Schuurs et al.) teaches a method for detecting HBsAg antibodies which utilizes enzyme labeled antibodies in a sandwich format.

Prior methods of detecting anti-HBc also include the use of radioimmunoassay (RIA) in a competitive format, as described in U.S. Patent No. 4,495,295 (Neurath). A direct RIA, as well as a competitive RIA, for anti-HBc is described by Overby, L.R. et al. in Presbyterian St. Lukes Med. Bull. (1976), 15:83-92. U.S. Patent No. 4,012,494 (Ling) teaches a direct RIA for hepatitis associated antibodies. The direct methods described by Overby et al. and Ling do not utilize a mixture of antibodies specific for human IgG and IgM. Since both IgM and IgG type anti-HBc is found in human sera or plasma, the methods taught by Overby et al. and Ling can lead to false negative results.

A direct or non-competitive immunoassay for an antibody of interest uses more wash steps than the competitive format and does not involve competition between labeled and unlabeled antibody for antigen binding sites. Rather, the antibody of interest is permitted to freely bind to an insolubilized antigen and then labeled antibodies specific for the bound antibody are permitted to react therewith. The amount of label detected is proportional to the bound antibody of interest. It is believed that direct binding, non-competitive immunoassays have a lower incidence of false positive results than do competitive immunoassays, possibly because of the additional wash steps used in the non-competitive format.

Monoclonal antibodies specific for HBcAg have been described by Waters, J.A. et al., in J. Med. Virol. (1986), 19:79-86. A competitive inhibition assay for the detection of anti-HBc using radiolabeled or peroxidase labeled forms of these monoclonal antibodies was described. Other workers have raised monoclonal antibodies against recombinantly produced HBcAg, which was synthesized in E. coli (Ferns, R.B. et al., J. Med. Virol. (1986), 19:193-203). The assay described was also a competitive type assay. The production of monoclonal antibodies specific for HBcAg resulted in the discovery that the human anti-HBc response is primarily directed toward a single immunodominant immunogenic epitope.

Summary of the Invention

The present invention provides a direct, non-competitive method for qualitatively detecting Hepatitis B core antibody (anti-HBc) in a biological fluid. The method comprises contacting the biological fluid to be tested with an insoluble surface coated with Hepatitis B core antigen (HBcAg) and permitting any anti-HBc in the sample to bind to the HBcAg. Following incubation and a wash step, the insoluble surface is then contacted with a mixture of a labeled antibody specific for human IgG and a labeled antibody specific for human IgM. Both antibodies may have the same type of label attached thereto or they may have different labels. Use of the mixture of antibodies avoids false negative results by providing a labeled antibody specific for IgG-anti-HBc and/or IgM-anti-HBc. If the biological sample only contains one of the anti-HBc types it will still be detected and a false negative result will be avoided. Following an incubation and wash step, the presence of the label bound to the insoluble surface indicates the presence of anti-HBc in the sample.

In a preferred embodiment, the label is an enzyme conjugated to the antibodies. In this embodiment, after the final incubation and washing steps, the insoluble surface is contacted with an an enzyme-substrate solution. The enzyme-substrate solution is developed and the optical density of the enzyme-substrate solution is measured. A value greater than a predetermined cutoff value indicates the presence of anti-HBc in the sample.

In the present method, anti-HBc is permitted to freely bind to the insolubilized HBcAg, i.e., there is no interference in binding to the antigenic epitope from a competing measured amount of labeled anti-HBc added by the investigator. Thus, non-competitive binding of anti-HBc is permitted.

The present invention also provides a method for confirming the presence of an antibody of interest in a biological fluid which has tested positive for the antibody in a first immunoassay. This confirmatory method comprises first providing an insoluble surface coated with an antigen for which the antibody of interest is specific. Then, the insoluble surface is contacted with an unlabeled antibody (monoclonal or polyclonal of non-human origin) specific for the antigen. As control, the insoluble surface is contacted with either diluent alone or with an unlabeled antibody (monoclonal or polyclonal of non-human origin) which lacks reactivity to the solid phase antigen. In both cases, the insoluble surface is also contacted, either simultaneously or subsequently, with the biological fluid to be tested. Following a wash step, the surface is contacted with at least one labeled antibody specific for the antibody of interest. Preferably, the labeled antibody is a mixture of a labeled antibody specific for human IgG and a labeled antibody specific for human IgM. Finally, the activity of the label bound to the insoluble surface is measured. As will be explained hereinafter, a value lower than that obtained in the absence of added unlabeled anti-HBc confirms the presence of the antibody of interest. The format of the confirmatory method can be different than the format of the first positive immunoassay. However, it is preferred that they be the same. Thus, if the first immunoassay is an ELISA format, then it is preferred that the confirmatory method also be an ELISA.

In a preferred embodiment of the confirmatory assay, the label is an enzyme. After incubation and washing, the surface is contacted with an enzyme-substrate solution. Finally, the optical density of the enzyme-substrate solution is measured. A value lower than that obtained in the absence of unlabeled anti-HBc confirms the presence of anti-HBc.

The present methods provide the advantages of excellent sensitivity, specificity and capability for confirmation. The present methods also provide a clear distinction between reactive and non-reactive samples by significantly reducing the possibility of false positives.

## EP 0 304 238 A2

Detailed Description of the Invention

The present invention provides a method for detecting an antibody of interest in a biological fluid suspected of containing the antibody. The method comprises contacting a first aliquot of said fluid with an insoluble surface coated with an antigen for which the antibody of interest is specific. Next, the insoluble surface is contacted with at least one labeled antibody specific for the antibody of interest. Preferably, the labeled antibody is a mixture of a labeled antibody specific for human IgG and a labeled antibody specific for human IgM. The activity of the label bound to the insoluble surface is measured in order to obtain a first value indicating the presence of the antibody of interest. Once a sample has tested positive, i.e. indicating the presence of the antibody of interest, the method is continued in order to confirm the presence of the antibody of interest. The first two contacting steps are repeated on a second aliquot of the biological fluid, except that when the aliquot is contacted with the insoluble surface it is contacted (prior to or simultaneously with) with an excess amount of an unlabeled antibody of non-human origin specific for the antigen. The unlabeled antibody is capable of blocking one or more of the epitopes on the antigen. Preferably, substantially all of the epitopes are blocked. The second aliquot is also contacted with the insoluble surface in the absence of unlabeled antibody of non-human origin specific for the antigen. The labeled antibody specific for the antibody of interest is added to the insoluble surface as before. And as before, the activity of the label on the insoluble surface is measured. However, this time, a value lower than the value obtained in absence of unlabeled antibody confirms the presence of the antibody of interest.

It is an important aspect of the present invention that the unlabeled antibody of non-human origin be capable of blocking (i.e. binding to) a sufficient number of epitopes on the antigen so that a detectably lower signal is obtained in the confirmatory assay. Less than substantially all of the epitopes may be blocked in order to achieve a useful confirmatory test. Preferably, substantially all of the major epitopes on the antigen are blocked. Preferably, the antigen has a single immunodominant epitope, such as exists on the HBcAg. In the case of an antigen with a single immunodominant epitope, a monoclonal antibody specific for that epitope or polyclonal sera raised against that epitope may be used as the unlabeled antibody. If the antigen has more than one epitope, or a limited number of well-defined epitopes, then an antibody of polyclonal origin or a mixture of monoclonal antibodies rather than a single monoclonal would be required to be used as the unlabeled antibody. The goal in such a case is to block a sufficient number of human antibodies from attaching to the multiple epitopes on the antigen. Thus, non-human polyclonal derived antibodies capable of blocking human antibodies from attaching to a multi-epitopic antigen could be used as the unlabeled antibody.

Use of the present confirmatory method allows for the identification of false positive results and the confirmation of weak positive results. False positive results may be caused by anything which can attach to or absorb to the insoluble surface (other than anti-HBc) which is capable of binding the labeled IgG specific or IgM specific antibodies. For example, nonspecific binding of any human IgG or IgM antibody to the insoluble surface will result in a false positive result. Also, if the insolubilized antigen is not pure and is contaminated with material that is capable of reacting with serum immunoglobins, a false positive result will be obtained.

If a positive result is obtained in a first immunoassay, the assay is run a second time except that an inhibiting amount of unlabeled antibody is added. The two assays are conducted identically as to all parameters, except for the addition of the unlabeled antibody. If no inhibition of binding of the labeled antibodies occurs, then the sample was a false positive. However, if there is inhibition of binding so that the label activity is substantially lower than in the absence of unlabeled anti-HBc or, in the extreme, if it is zero (i.e. 100% inhibition), then the sample is a true positive.

The present invention requires the use of labeled antibodies. The labeled antibodies, including the IgG specific and IgM specific antibodies of the present invention, may be labeled with any type of molecule which can be detected and which can be attached to or covalently bound to the antibody without negatively affecting its antigen binding activity. The label must have an activity or signal which can be directly or indirectly detected or measured. An example of a label which can be indirectly measured is a ligand, such as biotin, which can bind to another molecule, such as avidin, which is labeled with a detectable molecule. Suitable detectable labels are enzyme molecules which can cause a color change in a solution in the presence of an enzyme substrate, fluorescent molecules, radioactive molecules, chemiluminescent molecules or light scattering particles, such as colloidal gold. The labels may be the same or different on the IgG specific and IgM specific antibodies. Preferably, the labels are the same.

In another aspect, the present invention provides a novel method for detecting anti-HBc in which specific binding of anti-HBc to HBcAg on a solid phase is measured using a mixture of labeled anti-human IgG and IgM antibodies. The assay is described for that embodiment wherein the IgG and IgM antibodies

4

are enzyme labeled, however, as would be apparent to one skilled in the art, use of other labels is within the scope of the present invention.

The assay procedure for detecting anti-HBc is a 3-stage test which is preferably carried out in a plastic microwell coated with HBcAg. In the first stage, a biological fluid, such as serum or plasma, is diluted directly in the test well and incubated for a specified length of time. If anti-Hbc is present in the specimen, insolubilized antigen-antibody complexes will be formed on the solid phase as a result of immunoreaction and binding of anti-HBc to HBcAg. If anti-HBc is not present, complexes will not be formed and the unbound serum/plasma immunoglobulins will be removed in a washing step.

In the second stage of the assay, antibody-enzyme conjugate is added to the test well. The antibody conjugate is a mixture of two different antibodies. One antibody is specific for human IgG and the other is specific for human IgM. Each respective conjugate will bind specifically to the IgG-anti-HBc or IgM-anti-HBc portion of the insolubilized antigen-antibody complexes. If antigen-antibody complexes are not present, the unbound conjugates will be removed by washing. The two different types of anti-globulin antibodies are used because both IgM and IgG anti-HBc can be found in human sera or plasma. Thus, if the biological sample only contains anti-HBc of the IgG type, only the IgG antibody-conjugate will bind to the solid phase. Similarly, if the biological sample only contains anti-HBc of the IgM type, only the IgM antibody-conjugate will bind. If the biological sample contains anti-HBc of both the IgG and IgM types, then both the IgG and IgM antibody-conjugates will bind to the solid phase. If both types of antibodies were not used, false negative results (i.e., wrongly diagnosing a sample as not containing anti-HBc) could be obtained.

Quantitatively, antibodies of the IgG class constitute the most important group of antibodies present in human serum. Antibodies of the IgM class are present in the early stages of an infection, so that determination of antibodies of this class is important for the early detection of an infectious disease. Anti-HBc antibodies of the IgE, IgA or IgD classes are not found to any appreciable extent in human serum and, therefore, need not be taken into consideration in the present method.

The mixture of antibodies specific for human IgM and IgG antibodies is required in the detection of anti-HBc because the assay is non-competitive. The non-competitive format relies on the measurement of the direct binding of the antibody of interest. In a competitive format, it does not matter whether an IgM or an IgG antibody type is present in the biological specimen since the purpose of the antibody is to block the labeled antibody from binding to the immobilized antigen.

In the third stage of the assay, an enzyme substrate is added to the test well. If bound conjugate is present, the substrate will be oxidized, resulting in a colored end product. A suitable reaction terminating compound, such as sulfuric acid, is then added to stop the reaction. The optical density (or absorbance) of the enzyme substrate solution is then measured. The color intensity obtained is directly proportional to the amount of bound conjugate and therefore to the amount of specific IgG and/or IgM antibody present in the specimen. The color intensity may be measured with a microtitration plate reader.

If the sample tested gives a positive response for anti-HBc, this result may be confirmed by the confirmatory assay of the present invention. The confirmatory assay involves repeating all of the steps in the first three stages of the assay on a fresh aliquot of the sample as previously described above. However, when the aliquot is initially contacted with the insoluble surface coated with HBcAg, the aliquot is additionally contacted with an excess of unlabeled antibody, which may be monoclonal or polyclonal of non-human origin, specific for HBcAg. As control, the fresh aliquot is also contacted with the insoluble surface in the absence of unlabeled anti-HBc of non-human origin.

The unlabeled anti-HBc antibody must be of non-human origin in order to prevent reaction with the human IgG specific and IgM specific antibodies added in the subsequent step. If of monoclonal origin, the unlabeled antibody may be murine or rat derived. If of polyclonal origin, the unlabeled antibody may be derived from any suitable non-human source such as rabbit or goat anti-sera. Addition of the unlabeled antibody results in competition between the unlabeled antibody and anti-HBc in the sample for binding sites on HBcAg. The result is a decrease in binding of serum/plasma anti-HBc because the antigen epitope is substantially blocked. Since less sample anti-HBc is bound to the insoluble HBcAg, less anti-IgG/anti-IgM will bind. The optical density of the enzyme-substrate solution is measured as before, however, a value lower than the value obtained in the absence of unlabeled anti-HBc confirms the presence of anti-HBc. Although part of the confirmatory assay involves a competitive step between unlabeled antibody and antibody in the sample for HBcAg, the assay, considered as a whole, is still a direct, non-competitive assay. This is so because the assay involves measuring the amount of anti-HBc from the sample which is bound directly to the immobilized antigen.

The HBcAg used in the present invention may be recombinantly produced or obtained from purification of natural sources. Recombinantly produced HBcAg provides the advantages of a relatively unlimited and potentially pure source of antigen. Additionally, chemically synthesized fragments of HBcAg which function in the same manner as the entire antigen molecule may be used. Previously, production of HBcAg necessitated a complicated purification procedure using either Hepatitis B virus-infected liver or human serum containing the complete Hepatitis B virion as the starting material. Methods of producing HBcAg by recombinant DNA techniques are known in the art and described, for example, in Stahl, S. et al., Proc. Natl. Acad. Sci. USA (1982), 79:1606-1610; Edman, J.C. et al., Nature (1981) 291:503-506; and Miyanohara, A. et al., J. Virol. (1986) 59: 176-180.

The insoluble surface to which the HBcAg is immobilized may be any polymeric, silicaceous or polysaccharide material capable of absorbing or covalently binding the HBcAg. Plastic materials, such as polystyrene, are preferred polymeric materials. The insoluble surface may take the form of a flat surface or of beads or microparticles. It is preferred that the surface is the surface of a plastic microtitration well.

In that embodiment which uses enzyme labeled antibodies, the enzyme conjugated to the antibodies may be any enzyme which is capable of attaching to the antibody and reacting with a substrate to produce a color change in the enzyme-substrate solution. The enzyme conjugate consists of antibodies linked to one or more enzyme molecules. Such linking may be achieved either by direct condensation (covalent binding) or by using internal binding molecules in accordance with methods known to those skilled in the art. Suitable enzymes are peroxidase, such as horseradish peroxidase, and alkaline phosphatase. If the enzyme is horseradish peroxidase, a suitable substrate is o-phenylenediamine (OPD). If the enzyme is alkaline phosphatase, a suitable substrate is P-nitrophenyl phosphate.

The antiglobulin antibodies (anti-IgG and anti-IgM) used with the present method may be either monoclonal antibodies or antibodies of polyclonal origin. If a polyclonal serum is used it may either be specific for total human immunoglobulins or separately specific for human IgG and human IgM. Polyclonal antibodies can be prepared by methods known in the art, such as those described in Benedict, A.A. et al., "Production of Antiserum", Methods in Immunology 1:197-306 (1967). It is preferred that monoclonal antibodies be used in the present method because they are specific for a single epitope and therefore provide more reproducible results. Methods of producing monoclonal antibodies are known in the art and may be obtained by reference to Yelton, D.E. et al., Ann. Rev. Biochem (1981) 50:657-80. A method for producing monoclonal antibodies specific for HBcAg is taught in U.S. Patent No. 4,271,145 (Wands et al).

The antibody detected by the confirmatory method of the present invention may be any antibody which is specific for an antigen having a single epitope or a limited number of well defined epitopes. Preferably, the number of epitopes is no greater than five. Digoxin, thyroxine and vitamin B-12 are known to have single epitopes. Human chorionic gonadotropin (HCG) has two major well defined epitopes.

The following examples are presented to illustrate the subject invention. The invention is not to be considered limited by these examples but only by the appended claims.

## EXAMPLE 1

Recombinantly produced HBcAg was purchased from Chiron Corporation (Emeryville, CA). A 0.250 microgram/ml solution of the antigen was prepared in 0.01M sodium borate buffer, pH 9.3. Polystyrene microtitration wells (IMMULON II; Dynatech, Inc.) were coated with the antigen solution overnight at 4°C. The antigen nonspecifically absorbed to the surface of the wells. Nonabsorbed material was aspirated away. The wells were then countercoated with bovine serum albumin (10% in phosphate buffered saline) and dried following aspiration of the countercoat.

The HBcAg-coated microwell strips were assembled into a microwell strip holder. A negative control and a positive control were included in the assay run. Using a multi-channel micropipette, 200 microliters of specimen diluent was added to all wells except for the substrate blank. The specimen diluent was a phosphate-buffered saline solution with protein stabilizers containing 0.02% thimerosal as preservative. 10 microliters of the plasma/serum sample was then added to the appropriate wells. The microwell strip holder was covered with a plate cover and incubated at 37°C for 60 minutes. The wells were then aspirated with an aspirator-washer device and washed five times with a PBS-polysorbate 20 solution.

200 microliters of a monoclonal antibody conjugate mixture was added to each well. The mixture contained murine monoclonal antibodies for anti-human IgG (specific for gamma chain) and anti-human IgM (specific for mu chain), each covalently conjugated to horseradish peroxidase (HRP). The conjugated antibodies were prepared by standard techniques. The antibody mixture additionally contained protein stabilizers and was preserved with 0.02% thimerosal. The microwell strip holder was covered with a plate cover and incubated at 37°C for 60 minutes. Each well was then washed as previously described.

200 microliters of an OPD substrate solution was added to each well. The OPD solution was a solution of 0PD.2-CL in citrate-buffer containing 0.0125% hydrogen peroxide. The wells were incubated at room temperature (15°C - 30°C) in the dark for 30 minutes. The reaction was terminated by adding 50 microliters of 4N sulfuric acid to all wells.

The optical density (O.D.) of the controls and samples was then measured using a spectrophotometer with a wavelength set at 490 ± 2 nanometers. Specimens with absorbance values less than the cutoff value were considered negative (non-reactive). The cutoff value was determined by adding a predetermined value (e.g., 0.4) to the optical density of the negative control. The predetermined value was determined by analyzing large numbers of positive and negative samples and then choosing a cutoff value which provided the highest sensitivity and specificity. Specimens with absorbance values equal to or greater than the cutoff value were considered reactive.

Table 1 provides the results of the anti-HBc antibody assay for 7 patients. The negative control utilized was pooled normal human sera lacking antibodies to HBc. The positive control utilized was a diluted, core antibody positive serum sample. Specimens BB10, BB12 and BB14 contained anti-HBc antibodies and are from patients who have or have had hepatitis B. Specimens N2, N3 and N5 are from normal human donors lacking anti-HBc antibodies. Specimen WAD25 is a false positive sample.

Table 1

| Anti-HBc Antibody Assay | | |
|---|---|---|
| Specimen | O.D. | Reactivity |
| N2 | 0.211 | - |
| N3 | 0.122 | - |
| N5 | 0.160 | - |
| BB10 | 1.806 | + |
| BB12 | 1.750 | + |
| BB14 | 1.818 | + |
| WAD25 | 1.165 | + |

EXAMPLE 2

The samples which had tested positive in Example 1 were re-tested by the confirmatory method of the Present invention to confirm the presence of anti-HBc. The procedure was exactly the same as that utilized in Example 1 except that when the aliquot of the sample to be tested was added to the microtiter well, the aliquot was simultaneously contacted with an an excess amount of an unlabeled monoclonal antibody (diluted in specimen diluent) specific for HBcAg and diluent in parallel. Monoclonal anti-HBc (29C2A10) was diluted in diluent to a final concentration of 100 micrograms/ml before adding to the well. Optionally, the aliquot of the sample may be diluted so as to reduce the effective concentration of any anti-HBc in the sample. Each well was then treated as before with the same incubation and washing steps. When the optical density of the enzyme-substrate solution was measured, a value lower than the value obtained in the diluent only well confirmed the presence of anti-HBc in the biological sample.

Table 2 provides the results of the anti-HBc confirmatory assay for the specimens which previously tested positive.

Table 2

| Anti-HBc Antibody Confirmatory Assay | | | |
|---|---|---|---|
| Specimen | O.D. in presence of: | | % Inhibition |
| | Diluent | Anti-HBc | |
| BB10 | 1.893 | 0.106 | 110 |
| BB12 | 1.582 | 0.130 | 111 |
| BB14 | 1.761 | 0.144 | 109 |
| WAD25 | 0.848 | 0.965 | <0 |

Percent inhibition was determined using the following formula:

$$\frac{(D-N) - (H-N)}{(D-N)} \; X \; 100$$

where,

D = O.D. of the Diluent
N = O.D. of the Negative Control
H = O.D. of the anti-HBc

The sample designated WAD25 had a percent inhibition of less than zero and, therefore, was confirmed to be a false positive.

The invention has been described herein with reference to certain preferred embodiments and Examples. However, since obvious variations will appear to those skilled in the art, the invention is not to be considered limited thereto.

**Claims**

1. A method for detecting an antibody of interest in a biological fluid suspected of containing the antibody, comprising:

a) contacting a first aliquot of said fluid with an insoluble surface coated with an antigen for which the antibody of interest is specific;

b) contacting said surface with at least one labeled antibody specific for the antibody of interest;

c) measuring the activity of said label on said insoluble surface to obtain a first value indicating the presence of said antibody of interest;

d) repeating steps (a)-(b) on a second aliquot of said fluid except that in step (a) the fluid is additionally contacted with an unlabeled antibody of non-human origin specific for the antigen, wherein the unlabeled antibody blocks one or more of the epitopes on the antigen; and

e) measuring the activity of said label bound to said insoluble surface, wherein a value lower than the value obtained in step (c) in the absence of the unlabeled antibody confirms the presence of the antibody of interest.

2. The method of claim 1, wherein the labeled antibody specific for the antibody of interest is a mixture of a labeled antibody specific for human IgG and a labeled antibody specific for human IgM.

3. The method of claim 1 or claim 2, wherein step (d) additionally comprises running a control sample in parallel by separately contacting the insoluble surface with either diluent without the unlabeled antibody or with an unlabeled antibody which lacks reactivity with the insolubilized antigen.

4. The method of any preceding claim, wherein the unlabeled antibody blocks a sufficient number of epitopes on the antigen in order to detectably reduce the binding of the antibody of interest.

5. A non-competitive method for detecting the presence of Hepatitis B core antibody (anti-HBc) in a biological fluid, comprising:

a) contacting said fluid with an insoluble surface coated with hepatitis B core antigen (HBcAg) and permitting anti-HBc in said sample to non-competitively bind to said HBcAg;

b) contacting said surface with a mixture of a labeled antibody specific for human IgG and a labeled antibody specific for human IgM; and

c) detecting the presence of said label bound to said insoluble surface, thereby detecting the presence of anti-HBc in said sample.

6. A non-competitive method for detecting the presence of Hepatitis B core antibody (anti-HBc) in a biological fluid, comprising:

a) contacting said fluid with an insoluble surface coated with hepatitis B core antigen (HBcAg) and permitting anti-HBc in said sample to non-competitively bind to said HBcAg;

b) contacting said surface with a mixture of an enzyme conjugated antibody specific for human IgG and an enzyme conjugated antibody specific for human IgM;

c) contacting said surface with an enzyme-substrate solution; and

d) measuring the optical density of the enzyme-substrate solution.

7. A method for detecting hepatitis B core antibody (anti-HBc) in a biological fluid, comprising:

a) contacting a first aliquot of said fluid with an insoluble surface coated with hepatitis B core antigen (HBcAg);

b) contacting said surface with a mixture of an enzyme conjugated antibody specific for human IgG and an enzyme conjugated antibody specific for human IgM;

c) contacting said surface with an enzyme-substrate solution;

d) measuring the optical density of the enzyme-substrate solution to obtain a first value indicating the presence of anti-HBc;

e) repeating steps (a)-(c) on a second aliquot of said fluid except that in step (a) the fluid is additionally contacted with an unlabeled antibody of non-human origin specific for HBcAg; and

f) measuring the optical density of the enzyme-substrate solution, wherein a value lower than the value obtained in step (d) confirms the presence of anti-HBc.